(19) Europäisches Patentamt
European Patent Office
Office européen des brevets

(11) Numéro de publication: **0 066 485**
**B1**

(12) # FASCICULE DE BREVET EUROPEEN

(45) Date de publication du fascicule du brevet: **22.08.84**

(51) Int. Cl.[3]: **C 07 C 11/02**, C 07 C 5/27, C 07 C 11/09 // B01J23/44

(21) Numéro de dépôt: **82400823.9**

(22) Date de dépôt: **04.05.82**

(54) **Procédé d'isomérisation d'oléfines.**

(30) Priorité: **21.05.81 FR 8110312**

(43) Date de publication de la demande: **08.12.82 Bulletin 82/49**

(45) Mention de la délivrance du brevet: **22.08.84 Bulletin 84/34**

(84) Etats contractants désignés: **BE DE GB IT NL**

(56) Documents cités:
**FR - A - 913 756**
**FR - A - 1 247 782**
**US - A - 2 589 189**

(73) Titulaire: **INSTITUT FRANCAIS DU PETROLE, 4, Avenue de Bois-Préau, F-92502 Rueil-Malmaison (FR)**

(72) Inventeur: **Juguin, Bernard, 46, Avenue du Stade, F-92500 Rueil-Malmaison (FR)**
Inventeur: **Miquel, Jean, 5, rue Fragonard, F-75017 Paris (FR)**

**EP 0 066 485 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

Les additifs les plus économiques utilisés pour améliorer le pouvoir antidétonant des carburants sont des dérivés alkylés du plomb mais, du fait de la toxicité du plomb, leur emploi tend à diminuer. De plus, on sait que, dans les gaz de combustion des carburants, la teneur élevée en produits polluants tels que les hydrocarbures résiduels, l'oxyde de carbone et les oxydes d'azote, exige d'utiliser en général des pots catalytiques en vue d'abaisser cette teneur en produits polluants; ces pots catalytiques contiennent des catalyseurs appropriés renfermant très souvent, parmi leurs constituants, au moins un métal du groupe VIII de la classification périodique des éléments, ce métal étant, en général, le platine ou un autre métal noble de la famille du platine. Or, le plomb contenu dans les additifs de carburants et transporté par les gaz de combustion empoisonne très rapidement les catalyseurs des pots catalytiques en rendant ces derniers inefficaces pour l'utilisation qu'on leur demande. On a donc essayé des dérivés métalliques autres que les dérivés du plomb, par exemple le méthylcyclopentadiénylmanganèsetricarbonyle (MMT), mais ce produit s'est montré être un facteur de pollution non négligeable.

Ces divers problèmes ont donc conduit les raffineurs à produire des essences ou des constituants d'essence exempts d'agents antidétonants à base de plomb (plomb tétraéthyle, par exemple) mais qui, malgré tout, ont un nombre d'octane élevé.

Jusqu'ici l'essence sans plomb produite dans le monde provient préférentiellement des sources suivantes:

– reformage catalytique à haute sévérité du naphta,

– alkylation à l'isobutane des coupes $C_3$-$C_4$ de craquage catalytique, de craquage thermique, de coking, de visbreaking et de steam-cracking, contenant des oléfines.

L'essence sans plomb provenant du reformage catalytique à haute sévérité n'est pas idéale du point de vue de la pollution et de la santé publique. Elle contient en effet du benzène dont la vapeur s'est révélée très toxique.

L'alkylation, par contre, donne une essence satisfaisante tant du point de vue de l'écologie que du point de vue technique pur du moteur.

Malheureusement, la mise en œuvre de cette voie est essentiellement freinée en raison d'un manque d'isobutane.

En effet, la réaction entre l'isobutane et une oléfine $C_3$ ou $C_4$ est équimoléculaire; le calcul montre qu'il faut théoriquement 1,38 kg d'isobutane pour 1 kg de propylène ou 1,035 kg d'isobutane pour 1 kg de butènes.

Or, les coupes oléfiniques provenant, par exemple, du craquage à la vapeur (steam-cracking) et du craquage catalytique, présentent, en règle générale, un grave déséquilibre en ce qui concerne la teneur en isobutane qui est très insuffisante pour satisfaire la stœchiométrie évoquée ci-dessus. Par exemple, une coupe typique provenant d'un craquage catalytique présente la composition suivante en pour-cent en poids:

| | |
|---|---|
| propène: | 25,00 |
| propane: | 8,35 |
| isobutane: | 23,35 |
| isobutène: | 10,65 |
| n-butène-1: | 6,65 |
| n-butène-2: | 18,00 |
| n-$C_4$-(n-butane): | 8,00 |

Un calcul simple montre que la proportion d'isobutane est à peine le tiers de la proportion stœchiométrique d'oléfines.

Ce problème du déséquilibre des coupes $C_3$-$C_4$ est bien connu. Par exemple, le brevet US N° 3758628 propose d'y remédier en juxtaposant une unité d'hydrocraquage à une unité de craquage catalytique. Mais on assiste actuellement à une stagnation ou même à une réduction du nombre et de la capacité des unités d'hydrocraquage existantes. En outre, l'hydrocraquage est une opération coûteuse qui fournit de nombreux produits autres que l'isobutane et dont la valorisation n'est pas toujours possible.

On verra que le présent procédé selon l'invention permet d'utiliser avec rentabilité les coupes $C_3$-$C_4$ que forment les effluents de craquage catalytique ou de craquage à la vapeur (steam-cracking) ou de coking ou de craquage thermique ou de visbreaking.

En effet, on a assisté, ces dernières années, à des tentatives d'incorporation d'alcools d'éthers, dans l'essence soit pour améliorer l'indice d'octane, soit pour remédier à la pénurie de pétrole, soit pour d'autres motifs. De telles tentatives sont décrites, par exemple, dans les brevets US N° 3726942 et FR N° 2063939.

Ainsi, parce qu'il améliore l'indice d'octane des essences, le méthanol est l'un des additifs les plus intéresants.

Mais un autre additif intéressant est l'éther méthyltertiobutylique (ou méthyltertiobuthyléther (MTBE) en raison de ses propriétés antidétonantes qui lui permettent d'améliorer la qualité des essences commerciales, avec obtention d'un indice d'octane plus élevé que celui obtenu par l'utilisation du méthanol. En outre, le méthyltertiobutyléther (MTBE) possède un pouvoir calorifique plus élevé que celui du méthanol: 8395 kcal/kg (soit 4,18 × 8395 kJ/kg) pour le MTBE contre 4764 kcal/kg (soit 4,18 × 4764 kJ/kg) pour le méthanol (en moyenne, le pouvoir calorifique d'un super carburant est 10200 kcal/kg soit 4,18 × 10200 kJ/kg). De plus, le MTBE ne donne pas lieu à des problèmes de démixtion en présence d'eau, comme c'est le cas pour le méthanol. En outre encore, la solubilité de l'eau dans le MTBE étant considérablement supérieure à celle de l'eau dans les hydrocarbures, il s'ensuit que l'ajout de MTBE améliore la tolérance à l'eau des carburants.

Le MTBE présente également d'autres avantages:

– son point d'ébullition correspond à celui des constituants de l'essence ayant les propriétés antidétonantes les plus faibles,

– sa tension de vapeur ne présente aucun inconvénient,

– il possède un point de congélation excellent,

– comme sa solubilité dans l'eau est relativement faible et comme il est complètement miscible dans tous les hydrocarbures, il s'ensuit que les chances sont faibles de voir se présenter des problèmes de séparation de phases dans les mélanges de carburants, même en présence d'eau.

Bref, le MTBE se révèle un additif très intéressant pour l'amélioration des qualités des essences. Ce produit est généralement obtenu à partir de l'isobutène et du méthanol selon la réaction équilibrée ci-dessous:

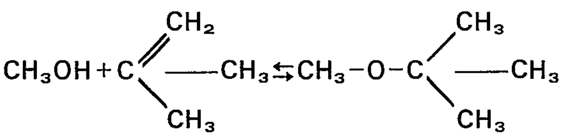

Une source d'isobutène est l'isobutène présent dans les coupes $C_3$-$C_4$ oléfiniques en provenance des effluents de craquage catalytique, de craquage à la vapeur, de coking, de visbreaking et de cracking thermique. Une autre source d'isobutène est encore la fraction provenant d'un effluent d'une unité de production du MTBE, fraction obtenue après avoir traité ledit effluent de façon à enlever le MTBE produit, extraire éventuellement les paraffines présentes et hydrogéner éventuellement le butadiène.

Il convient cependant de remarquer qu'une production de 100000 t/an de MTBE à partir par exemple d'une coupe $C_3$-$C_4$ de craquage à la vapeur (steam-cracking) équivaut à disposer d'une unité de craquge qui fournirait 500000 t/an d'éthylène, et que, dans les coupes $C_3$-$C_4$ oléfiniques en provenance d'un craquage catalytique, les teneurs en isobutène sont plus faibles encore que celles obtenues à partir d'un effluent de craquage à la vapeur.

Les possibilités limitées d'approvisionnement en isobutène sont donc incompatibles avec l'ampleur du développement de la production de MTBE.

On a alors proposé un nouveau mode d'obtention d'isobutène qui consiste non plus à isoler l'isobutène présent dans les coupes $C_3$-$C_4$ énumérées ci-dessus, mais à modifier la composition des $C_4$ de la coupe $C_3$-$C_4$ oléfinique, avec transformation, par isomérisation, de la totalité ou de la presque totalité des butènes contenus dans cette coupe $C_3$-$C_4$ en isobutène.

Beaucoup de procédés et beaucoup de catalyseurs ont été proposés à cet effet et notamment les catalyseurs à base d'alumines, et en particulier à base d'alumines activées (par exemple, l'alumine $\eta$ et l'alumine $\gamma$), d'alumines halogénées, de bauxite, d'alumines traitées avec les dérivés du bore ou du silicium ou du zirconium, de silices-alumines diverses, de phosphates plus ou moins complexes, d'acide phosphorique solide.

Le procédé selon l'invention est un perfectionnement de l'art antérieur et plus particulièrement des brevets suivants:

– USP N° 2471647 dans lequel on utilise un catalyseur à base d'alumine fluorée, où la teneur en acide fluorhydrique est comprise entre environ 0,5 et 10%,

– USP N° 2395274 où le catalyseur est de la bauxite et où l'on opère en présence de vapeur d'eau,

– USP N° 3558733 où le catalyseur à base d'alumine est réactivé par de la vapeur d'eau, sans qu'il y ait contact entre la vapeur d'eau et les oléfines que l'on isomérise,

– USP N° 3558734 où le catalyseur halogéné renferme 5 à 100 ppm d'eau environ,

– USP N° 2422884 où l'on opère en présence de vapeur d'eau (rapport molaire $H_2O/HC$ variant de 0 à 10) avec un catalyseur à base d'alumine borée.

Les résultats obtenus selon ces divers procédés laissent à désirer.

Ainsi, les catalyseurs à base d'alumine fluorée ne sont pas toujours, dans l'art antérieur, les meilleurs des catalyseurs d'isomérisation. Ils possèdent certes une très bonne activité mais, du fait de leurs propriétés acides, ces solides activent non seulement l'isomérisation, mais aussi les réactions secondaires indésirables de polymérisation et de craquage, qui sont la cause de la baisse de la sélectivité et de la diminution de la durée de vie du catalyseur. De tels catalyseurs imposent en outre une introduction continuelle de fluor pour compenser les pertes en fluor en cours de réaction, et ces catalyseurs donnent lieu également à des corrosions inhérentes aux dérivés fluorés.

Les catalyseurs utilisés présentent des inconvénients dont les plus importants sont une sélectivité faible, du fait de l'existence de réactions parasites comme le craquage et la polymérisation, et un manque de stabilité entraînant une diminution plus ou moins rapide du taux de conversion; en plus, ces catalyseurs sont difficiles à régénérer ou pas du tout régénérables.

En outre, pour obtenir avec ces catalyseurs des taux de conversion convenables, il faut utiliser des vitesses spatiales assez faibles, d'où la nécessité d'utiliser des réacteurs de plus grande capacité.

L'objet de l'invention remédie à tous ces inconvénients. Il consiste à opérer à l'aide d'un catalyseur à base d'alumine acidifiée par une quantité critique de silice et en présence de vapeur d'eau.

L'emploi d'eau ou de vapeur d'eau, dans l'art antérieur, ne s'est pas révélé apporter des améliorations suffisamment satisfaisantes pour l'isomérisation d'oléfines.

Or, on a maintenant constaté qu'il convenait d'utiliser un catalyseur à base d'alumine à laquelle on a ajouté une quantité critique de silice, à condition d'opérer en présence d'eau en quantité également critique.

Ainsi, la présence simultanée d'un catalyseur à base d'alumine et de silice ainsi que d'eau dans le milieu réactionnel inhibe les réactions secondaires de manière surprenante.

On obtient ainsi des taux élevés du produit désiré avec une excellente vitesse de transformation et des sélectivités très bonnes, avec l'avantage de pouvoir opérer à des températures plus faibles qu'à l'habitude, c'est-à-dire de travailler sous des conditions où l'équilibre thermodynamique est plus favorable.

Selon le procédé de l'invention, on peut indifféremment isomériser une seule coupe oléfinique $C_4$ en provenance d'un craquage ou toute autre réaction énumérée plus haut (après élimination de la coupe $C_3$) ou l'ensemble de la coupe oléfinique $C_3$-$C_4$, les hydrocarbures en $C_3$ n'étant pas touchés par l'isomérisation des $C_4$-(butènes) et ne gênant pas le processus de l'isomérisatgion. La coupe $C_3$-$C_4$ soumise dans sa totalité au traitement d'isomérisation peut également, sans inconvénient, contenir des hydrocarbures en $C_1$, $C_2$ et $C_5^+$ jusqu'à 20 atomes de carbone par molécule.

Mais, selon le procédé de l'invention, on peut isomériser d'autres hydrocarbures oléfiniques comportant 5 à 20 atomes de carbone par molécule possédant une struture à chaîne droite pour les transformer en hydrocarbures oléfiniques à structure ramifiée, ayant le même nombre d'atomes de carbone par molécule que l'oléfine dont ils proviennent. Ainsi on peut avantageusement transformer des pentènes en isopentènes ou des hexènes en isohexènes.

Les températures préférées que l'on utilise pour effectuer le présent procédé sont comprises entre 300 et 550° C, et plus particulièrement entre 400 et 500° C. Il va de soi que, à des températures plus élevées que celles indiquées ici, la réaction d'isomérisation est toujours possible mais, si elle est plus rapide qu'à température plus modérée, par contre apparaissent alors les réactions secondaires que l'on veut éviter, telles que le craquage, la déshydrogénation, la polymérisation, le coking. De plus, il faut insister sur le fait que la concentration en isooléfine (isobutène par exemple) à l'équilibre décroît avec l'élévation de la température. Mais, inversement, il ne convient pas non plus de vouloir trop diminuer la température de réaction, cela entraînant, d'une part, une vitesse de réaction plus faible et, d'autre part, une accélération de la réaction de polymérisation en dépit du fait que, généralement, la vitesse de polymérisation n'est pas favorisée par une diminution de la température. En opérant conformément à l'invention et de préférence dans le domaine de température préconisé, la réaction d'isomérisation a lieu à une vitesse suffisamment rapide, et on atteint rapidement des concentrations en isooléfines (isobutène par exemple) très proches de celles correspondant à l'équilibre thermodynamique, tout en conservant la vitesse des réactions indésirables à des niveaux assez faibles. Il va de soi que le choix précis de la température adéquate de réaction dépend du type d'oléfine à isomériser ainsi que du temps de contact et d'autres variables.

La réaction indésirable de polymérisation étant favorisée par un accroissement de la pression, on préfère travailler à des pressions assez faibles, généralement comprises entre 0,05 et 1 MPa et de préférence entre 0,08 et 0,4 MPa (1 MPa = 10 kg/cm²).

En général, on utilise une vitesse spatiale comprise entre 0,1 et 10 exprimée en volumes de charge oléfinique liquide par volume de catalyseur et par heure, et de préférence comprise entre 0,5 et 4 volumes d'oléfine à l'état liquide par volume de catalyseur et par heure.

Conformément à l'invention, on mélange dans la charge, ou on ajoute à la charge, par une ligne séparée, la quantité d'eau nécessaire au maintien de la bonne sélectivité du catalyseur. La quantité d'eau introduite dans le réacteur est choisie de façon à avoir dans la zone de réaction un rapport molaire

$$\frac{H_2O}{\text{hydrocarbures de la charge oléfinique}}$$

compris entre 0,1 et 10 et de préférence entre 0,5 et 3, et plus particulièrement entre 0,8 et 2,7.

Pour la préparation du catalyseur, on peut utiliser une alumine commerciale de type quelconque mais de préférence activée, choisie également de préférence parmi les alumines du type η ou γ à faible teneur en métaux alcalins et alcalino-terreux, par exemple contenant moins de 0,1% en poids de sodium et/ou d'autres métaux alcalins et/ou alcalino-terreux.

Cette alumine est généralement utilisée sous forme de billes ou d'extrudés ou de pastilles ou de concassés, de dimensions suffisantes pour laisser un passage relativement facile aux réactifs gazeux.

La surface spécifique de l'alumine utilisée peut avantageusement être comprise entre 10 et 350 m²/g, de préférence entre 50 et 250 m²/g, son volume poreux étant compris par exemple entre 0,4 et 0,8 cm³/g.

Cette alumine est activée par exemple par chauffage avec un courant d'air sec à une température comprise entre 350 et 550° C pendant plusieurs heures.

La silice peut être introduite dans l'alumine par un moyen quelconque sous la forme d'un dérivé de silicium soluble dans un solvant approprié.

On pourra par exemple imprégner l'alumine à sec avec une solution alcoolique de silicate d'éthyle, laisser le mélange en contact le temps nécessaire à une bonne diffusion du silicate d'éthyle à travers tout le volume poreux du solide.

Puis on chasse le solvant et ensuite on hydrolyse le silicate d'éthyle en présence de vapeur d'eau de manière que la silice formée se fixe sur la surface de l'alumine.

Ensuite, on peut, par exemple, sécher le solide que l'on calcine plus tard à une température comprise, par exemple, entre 550 et 650°C en présence d'air. La quantité de silice introduite est telle que le catalyseur fini ait une concentration en $SiO_2$ comprise entre 0,5 et 10% en poids, de préférence entre 1 et 6%, et plus particulièrement entre 2 et 4%.

Il convient néanmoins de signaler que le mode de préparation indiqué ci-dessus n'est pas limitatif; en effet, on peut aussi déposer la silice sur de

l'alumine se trouvant déjà placée dans la zone réactionnelle d'isomérisation.

On peut ainsi par exemple imprégner l'alumine en lit par introduction d'une solution alcoolique contenant la quantité nécesaire de silicate d'éthyle, chasser l'excès de solvant, procéder ensuite à l'hydrolyse, en présence de vapeur d'eau, suivie d'un séchage *in situ* et d'une calcination. L'introduction de la charge fait suite à cette préparation.

On peut aussi déposer la silice sur l'alumine en faisant passer à travers le lit d'alumine le silicate d'éthyle en phase vapeur, et effectuer ensuite les opérations auparavant décrites.

Une autre manière de procéder consiste à introduire le précurseur, contenant la silice à déposer, à l'état dissous dans la charge. Dans le but de faciliter la régénération du catalyseur usé en cours de réaction, notamment par combustion des produits fortemnt polymérisés et même du carbone qui auraient pu se déposer sur la surface du solide au cours de la réaction, il convient, conformément à l'invention, d'introduire à la surface du catalyseur un agent capable de faciliter la combustion de ces produits organiques. Comme agent activant la combustion, on peut utiliser tout métal ou oxyde ou tout autre dérivé de ce métal capable d'activer les réactions doxydation. A titre d'exemples non limitatifs de tels agents, on peut citer, par exemple, l'emploi d'au moins un métal choisi dans le groupe constitué par le manganèse, l'argent, le chrome, le palladium, le nickel, le cuivre et les oxydes de ces métaux.

Le palladium est un agent particulièrement indiqué pour activer ladite combustion des produits organiques.

Ainsi, l'ajout de palladium (et il en est de même avec les autres métaux cités plus haut) se traduit par une meilleure facilité de régénération du catalyseur, permettant notamment l'emploi d'une température plus douce qu'à l'habitude pour effectuer le démarrage de la régénération, la température de démarrage pouvant être de l'ordre de 200 à 250°C au lieu de 325 à 375°C. Si le catalyseur renferme du palladium, la concentration en oxygène exigée est moins importante. Cela se traduit par un front de flamme moins chaud qu'à l'habitude (ce front de flamme mesuré au thermocouple étant, sans palladium, de 400 à 450°C environ, correspondant à une température de 500 à 550°C environ dans la masse du grain du catalyseur ou de la bille de catalyseur). Ainsi, l'ajout de palladium entraîne une vitesse de combustion moindre, expliquant l'élévation moindre de la température au cours de la régénération. On ajoutera qu'un front de flamme moins chaud diminue également les dangers durant les manipulations que nécessite la régénération du catalyseur.

Le palladium peut être introduit sur le catalyseur, par exemple par imprégnation sous la forme d'un sel d'un autre composé contenant le palladium, pourvu que le produit soit soluble dans l'eau ou tout autre solvant utilisé.

On peut, par exemple, utiliser du nitrate de palladium.

La quantité de métal introduite varie entre environ 5 ppm et 2% environ en poids par rapport au catalyseur. Si l'on utilise du palladium, la quantité de palladium introduite est comprise entre 5 et 2000 ppm, de préférence entre 5 et 100 ppm. Avantageusement, on utilise 10 à 30 ppm de palladium.

A noter que les deux composés que l'on peut déposer sur l'alumine, à savoir la silice et un métal ou composé de métal additionnel, peuvent éventuellement être introduits en une seule imprégnation, ou en deux imprégnations successives avec un séchage et une calcination intermédiaires si on introduit d'abord le métal (par exemple le palladium), ou avec une hydrolyse suivie d'un séchage et calcination si on dépose la silice en premier.

Le catalyseur peut être utilisé sous la forme d'un lit fixe, d'un lit fluidisé ou d'un lit mobile.

Lorsque l'on uitlise un catalyseur sous la forme d'un lit mobile, un procédé particulier consiste à utiliser plusieurs réacteurs en série, placés par exemple côte à côte ou superposés: la charge circule successivement dans chaque réacteur suivant un écoulement axial ou radial. Le catalyseur frais est introduit en haut du premier réacteur où est introduite la charge fraîche; il s'écoule ensuite progressivement de haut en bas de ce réacteur d'où il est soutiré progressivement par le bas, et par tout moyen approprié (lift en particulier dans le cas de réacteurs disposés côte à côte), il est transporté en haut du réacteur suivant dans lequel il s'écoule progressivement également de haut en bas, et ainsi de suite jusqu'au dernier réacteur en bas duquel le catalyseur est également soutiré progressivement, puis envoyé dans une zone de régénération. A la sortie de la zone de régénération, le catalyseur est réintroduit progressivement dans le haut du premier réacteur, de manière à maintenir un niveau d'activité élevé et sensiblement constant à chaque point des zones catalytiques. Les divers soutirages de catalyseur sont effectués, comme indiqué ci-dessus, progressivement, c'est-à-dire soit périodiquement, soit en continu.

La régénération et l'ajustage du catalyseur s'effectuent par tout moyen classique connu qu'il est inutile de décrire ici.

Le catalyseur utilisé dans les exemples qui vont suivre a été préparé à partir d'une alumine commerciale fabriquée par Rhône-Progil et fournie sous forme de billes de 1 à 2 mm de diamètre. Cette alumine appartient au type $\gamma$ cubique; sa densité de grain est de 1,23, sa densité structurale de 3,27, son volume poreux total est de 0,51 $cm^3/g$ et sa surface spécifique est de 210 $m^2/g$.

La teneur en métaux alcalins de l'alumine est inférieure à 100 ppm et celle en alcalino-terreux est inférieure à 300 ppm.

On a imprégné 100 g de cette alumine, préalablement calcinée à 500°C, pendant 2 h avec une solution contenant:

7,2 $cm^3$ de silicate d'éthyle pur

2 $cm^3$ de solution aqueuse de nitrate de palladium à 0,1% de Pd, et

60 $cm^3$ d'alcool éthylique absolu.

On a laissé le tout en contact pendant 6 h à la température ambiante (19°C). Puis on a placé le tout dans une étuve en atmosphère humide (humidité relative de 90%) à 40°C pendant 12 h; ensuie on monte progressivement, en 1 h, la température à 95°C, toujours sous atmosphère humide (humidité relative de 95%) et on laisse à cette température pendant 12 h. Puis on porte le tout à 110° pendant 12 h sous atmosphère sèche.

Le produit sec est ensuite calciné à 630°C sous air.

Le catalyseur fini a la composition suivante (en poids):

3% de $SiO_2$
20 ppm de palladium et complément à 100% d'alumine.

*Exemple 1:*

On a placé une partie du catalyseur obtenu ci-dessus dans un réacteur à lit fixe.

A travers ce lit, on fait passer une charge d'hydrocarbures oléfiniques en $C_4$ dont la constitution est donnée dans le tableau I, conjointement avec de l'eau.

Les conditions opératoirse sont les suivantes:
Température: 450°C
Pression: 0,1 MPa (1 kg/cm²)
Volume de charge $C_4$ liquide par volume de catalyseur et par heure: 2
Rapport molaire $H_2O/HC$ de la coupe oléfinique: 1,35

Dans le tableau I, on donne également la composition du produit obtenu, en pour-cent en pondéral.

*Tableau I*

| | Charge (% en poids) | Produit (% en poids) |
|---|---|---|
| Méthane | — | 0,03 |
| Ethane + éthylène | — | 0,07 |
| Propane | — | — |
| Propène | 0,12 | 1,10 |
| Butane normal | 16,24 | 17,10 |
| Isobutane | 4,73 | 4,78 |
| Butène-1 | 10,14 } 77,81 | 14,12 } 53,52 |
| Butène-2 | 67,67 | 39,20 |
| Isobutène | 1,10 | 23,60 |
| Polymères | — | — |

Pour exprimer plus clairement les résultats, on utilisera les définitions suivantes:

$$\text{Conversion \%} = \frac{\Delta[\text{n-butènes}]}{\Sigma[\text{n-butènes (à l'entrée)}]}$$

$$\text{Sélectivité en \% isobutène} = \frac{\Delta[\text{isobutène}]}{\Delta\text{n-butènes}}$$

$$\text{Sélectivité en polymérisation \%} = \frac{\Sigma[C_5^+]}{\Delta[\text{n-butènes}]}$$

$$\text{Sélectivité en craquage \%} = \frac{\Sigma[C_1,C_2,C_3]}{\Delta[\text{n-butènes}]}$$

$$\text{Sélectivité en transfert d'hydrogène \%} = \frac{\Delta[C_4(\text{paraf})]}{\Delta[\text{n-butènes}]}$$

Les symboles repris ci-dessus ont les significations suivantes:

Δ[n-butènes] = Σn-butènes (à l'entrée) − Σn-butènes (à la sortie)

Δ[isobutène] = isobutène (à la sortie) − isobutène (à l'entrée)

$\Sigma(C_1, C_2, C_3)$ = Σ-hydrocarbures en $C_1$, $C_2$ et $C_3$ saturés et non saturés

$\Delta C_4$ (paraf) = Σ-butanes (sortie) − Σ-butanes (entrée)

$C_5^+$ = hydrocarbures possédant 5 atomes de carbone et plus.

La conversion globale des butènes-1 et -2 est de 31,5% (en poids).

Les sélectivités par rapport aux butènes transformés sont les suivantes (exprimées % en poids):
Sélectivité en isobutène = 91,9
Sélectivité en polymérisation = 0
Sélectivité en craquage = 4,9
Sélectivité en transfert d'hydrogène = 3,7

Rapport pondéral entre la totalité des butènes à la sortie et à l'entrée du réacteur (en %): 98,8.

Rapport pondéral entre la totalité de la coupe $C_4$ à la sortie et à l'entrée du réacteur (en %): 98,9.

Rapport pondéral isobutène/coupe $C_4$ (en %) (à la sortie du réacteur): 23,9.

Rapport pondéral (en %) isobutène/Σ-oléfines en $C_4$ (à la sortie du réacteur): 30,7.

Teneur en carbone du catalyseur après 10 h de marche: 0,4%.

*Exemple 2:*

On répète l'exemple 1 en modifiant ici la vitesse spatiale et le rapport molaire $H_2O/HC$.

Vitesse spatiale en volume de charge oléfinique liquide par volume de catalyseur et par heure: 1.

Rapport molaire $H_2O/HC$ de la coupe oléfinique: 2,2.

On redonne dans le tableau II la composition de la coupe $C_4$ oléfinique utilisée comme charge, et on y donne la composition du produit obtenu après isomérisation:

*Tableau II*

| | Charge (% en poids) | Produit (% en poids) |
|---|---|---|
| Méthane | — | 0,04 |
| Ethane + éthylène | — | 0,16 |
| Propane | — | 0,02 |
| Propène | 0,12 | 2,10 |
| Butane normal | 16,24 | 17,31 |
| Isobutane | 4,73 | 4,54 |
| Butène-1 | 10,14 } 77,81 | 11,80 } 46,00 |
| Butène-2 | 67,67 | 34,20 |
| Isobutène | 1,10 | 29,83 |
| Polymères | — | — |

La conversion globale des butènes-1 et -2, en poids, est de:

$$\left(\frac{77,81-46}{77,81}\times 100\right) = 40,9\%$$

Les sélectivités par rapport aux butènes-1 et -2 transformés sont les suivantes (exprimées en % poids):

Sélectivité en isobutène = 90,3
Sélectivité en polymérisation = 0
Sélectivité en craquage = 7,3
Sélectivité en transfert d'hydrogène = 2,8

Rapport entre la totalité des butènes à la sortie et à l'entrée du réacteur, en % pondéral: 96,1.

Rapport entre la totalité de la coupe $C_4$ à la sortie et à l'entrée du réacteur, en % poids: 97,8.

Rapport isobutène/coupe $C_4$, en % poids (à la sortie du réacteur): 30,5.

Rapport isobutène/$\Sigma$-oléfines en $C_4$, en % poids (à la sortie du réacteur): 39,3.

Teneur en carbone du catalyseur après 10 h de marche: 0,3%.

*Exemple 3* (comparatif):

On opère comme dans l'exemple 1 mais, dans le présent exemple, on introduit la coupe $C_4$-oléfinique à isomériser dans le réacteur sans addition d'eau.

Dans le tableau III, on donne la composition de la charge et celle du produit isomérisé.

*Tableau III*

| | Charge (% en poids) | Produit (% en poids) |
|---|---|---|
| Méthane | — | 0,39 |
| Ethane + éthylène | — | 1,2 |
| Propane | — | 0,35 |
| Propène | 0,12 | 8,2 |
| Butane normal | 16,24 | 17,9 |
| Isobutane | 4,73 | 8,4 |
| Butène-1 | 10,14 } 77,81 | 11,83 } 37,54 |
| Butène-2 | 67,67 | 25,71 |
| Isobutène | 1,10 | 19,90 |
| Polymères | — | 6,12 |

La conversion globale des butènes-1 et -2 est de (en poids):

$$100\times\frac{77,81-37,54}{77,81} = 51,7\%$$

Les sélectivités par rapport aux butènes-1 et -2 transformés sont les suivantes (exprimées en % poids):

Sélectivité en isobutène: 46,7
Sélectivité en polymérisation: 15,2
Sélectivité en craquage: 25,2
Sélectivité en transfert d'hydrogène: 13,2

Rapprot entre la totalité des butènes à la sortie et à l'entrée du réacteur, en % pondéral: 72,8.

Rapport entre la totalité de la coupe $C_4$ à la sortie et à l'entrée du réacteur, en % poids: 83,8.

Rapport isobutène/coupe $C_4$, en % poids à la sortie du réacteur: 23,7.

Rapport isobutène/$\Sigma$-oléfines en $C_4$, en % poids à la sortie du réacteur: 34,6.

Teneur en carbone du catalyseur après 10 h d'opération: 25,5%.

Cet exemple montre que l'absence d'eau entraîne une chute de la sélectivité ainsi qu'un dépôt prohibitif de carbone, entraînant ainsi une rapide désactivation du catalyseur.

On répète l'exemple avec injection d'une faible quantité de vapeur d'eau, le rapport molaire:

$$\frac{H_2O}{\text{HC de la charge oléfinique}}$$

étant égal à 0,05.

On obtient sensiblement les mêmes résultats que ci-dessus.

*Exemple 4:*

On traite du pentène-1 dans les conditions opératoires de l'exemple 1.

Parmi les produits soutirés de la zone de réaction, on trouve, en plus des pentènes linéaires, les isopentènes recherchés ainsi que des produits de craquage et de polymérisation.

Nous présentons ci-dessous les résultats obtenus:

Conversion % en poids: 34,5.

Les sélectivités du pentène transformés sont:

| | |
|---|---|
| en isopentènes (% poids): | 92,1 |
| en polymérisation: | 0 |
| en craquage (% poids): | 5,1 |
| transfert d'hydrogène (% poids): | 3,4 |

teneur en carbone du catalyseur après 10 h de marche (%): 0,5

*Exemple 5:*

On répète l'exemple 1 en utilisant un catalyseur renfermant 11% de silice au lieu de 3% dans l'exemple 1. La quantité de palladium est 20 ppm et le complément à 100% est de l'alumine. Les résultats figurent dans le tableau IV.

*Tableau IV*

| | Charge (% en poids) | Produit (% en poids) |
|---|---|---|
| Méthane | — | 0,06 |
| Ethane + éthylène | — | 0,03 |
| Propane | — | 0,06 |
| Propène | 0,12 | 2,40 |
| Butane normal | 16,24 | 17,50 |
| Isobutane | 4,73 | 4,80 |
| Butène-1 | 10,14 } 77,81 | 15,25 } 53,35 |
| Butène-2 | 67,67 } | 38,10 } |
| Isobutène | 1,10 | 21,60 |
| Polymères | — | 0,2 |

La conversion globale des butènes-1 et -2 est de (% en poids):

$$\frac{77,81-53,35}{77,81} \times 100 = 31,44$$

Les sélectivités par rapport aux butènes transformés sont les suivantes (exprimées en % en poids):

| | |
|---|---|
| Sélectivité en isobutène: | 83,81 |
| Sélectivité en polymérisation: | 0,82 |
| Sélectivité en craquage: | 9,93 |
| Sélectivité en transfert d'hydrogène: | 5,44 |

Rapport pondéral entre la totalité des butènes à la sortie et à l'entrée du réacteur (en %): 95.

Rapport pondéral entre la totalité de la coupe $C_4$ à la sortie et à l'entrée du réacteur (en %): 97,37.

Rapport pondéral isobutène/coupe $C_4$ (en %) (à la sortie du réacteur) (en %): 22,21.

Rapport pondéral (en %) isobutène/$\Sigma$-oléfines en $C_4$ (à la sortie du réacteur): 28,72.

Teneur en carbone du catalyseur après 10 h de marche: 0,42.

On note en comparant les exemples 1 et 5 que, avec une proportion trop forte de silice (exemple 5):

la conversion est équivalente dans les deux exemples,

la sélectivité en isobutène commence à diminuer dans l'exemple 5,

un début de polymérisation apparaît dans l'exemple 5,

le craquage augmente dans l'exemple 5.

On note encore que, dans l'exemple 5:

le transfert d'hydrogène augmente également,

le rapport butènes sortie/entrée diminue,

le rapport $\Sigma$-$C_4$ sortie/entrée diminue,

le rapport (sortie réacteur) isobutène/coupe $C_4$ diminue,

le rapport isobutène/oléfines-$C_4$ diminue, et

la teneur en carbone augmente.

## Revendications

1. Procédé d'isomérisation d'hydrocarbures éthyléniques linéaires comportant 4 à 20 atomes de carbone par molécule en hydrocarbures ramifiés éthyléniques à même nombre d'atomes de carbone par molécule que l'hydrocarbure linéaire dont ils proviennent, caractérisé en ce que l'on traite une charge d'hydrocarbures constituée au moins en partie d'hydrocarbures éthyléniques linéaires, en présence de vapeur d'eau et d'un catalyseur renfermant: a) de l'alumine, b) 0,5 à 10% en poids de silice, et c) au moins un métal ou composé de métal, ledit métal étant choisi dans le groupe constitué par le chrome, le palladium, le nickel, le cuivre, le manganèse et l'argent, la concentration en métal étant comprise entre 5 ppm et 2% en poids par rapport au catalyseur, le rapport molaire $H_2O$/hydrocarbures de la charge étant compris entre 0,1 et 10.

2. Procédé selon la revendication 1, dans lequel le rapport molaire $H_2O$/hydrocarbures éthyléniques linéaires à isomériser est compris entre 0,5 et 3.

3. Procédé selon la revendication 2, dans lequel le rapport molaire $H_2O$/hydrocarbures éthyléni-

ques linéaires à isomériser est compris entre 0,8 et 2,7, et dans lequel le catalyseur renferme 1 à 6% de silice.

4. Procédé selon l'une des revendications 1 à 3, dans lequel le métal est le palladium, la concentration en palladium étant comprise entre 5 et 100 ppm par rapport au catalyseur.

5. Procédé selon la revendication 4, dans lequel ladite concentration en palladium est comprise entre 10 et 30 ppm.

6. Procédé selon l'une des revendications 1 à 5, dans lequel on opère entre 300 et 550° C, sous une pression comprise entre 0,05 et 1 MPa et avec une vitesse spatiale, exprimée en volumes de charge liquide par volume de catalyseur et par heure, comprise entre 0,1 et 10, ledit procédé étant appliqué à la fabrication d'isobutène par traitement d'une charge choisie dans le groupe constitué par un effluent de craquage catalytique, un effluent de craquage à la vapeur, un effluent de coking, un effluent de craquage thermique, un effluent de visbreaking et une fraction en provenance d'un effluent d'une unité de MTBE (méthyltertiobutyléther), ladite charge étant constituée au moins en partie de butène-1 et de butène-2.

7. Procédé selon la revendication 6, effectué à une température comprise entre 400 et 500° C sous une pression comprise entre 0,08 et 0,4 MPa avec une vitesse spatiale, exprimée en volumes de charge liquide par volume de catalyseur et par heure, comprise entre 0,5 et 3.

8. Procédé selon la revendication 2, dans lequel le catalyseur renferme 2 à 4% en poids de silice.

## Patentansprüche

1. Verfahren zur Isomerisierung von linearen ethylenischen Kohlenwasserstoffen mit 4 bis 20 Kohlenstoffatomen pro Molekül in verzweigtkettige ethylenische Kohlenwasserstoffe mit der gleichen Anzahl von Kohlenstoffatomen pro Molekül wie der lineare Kohlenwasserstoff, von dem sie abstammen, dadurch gekennzeichnet, dass man eine Kohlenwasserstoffcharge, die zumindest teilweise aus linearen ethylenischen Kohlenwasserstoffen besteht, in Gegenwart von Wasserdampf und einem Katalysator behandelt, der a) Aluminiumoxid, b) 0,5 bis 10 Gew.-% Siliciumdioxid, und c) mindestens ein Metall oder eine Verbindung eines Metalls, das aus der Gruppe bestehend aus Chrom, Palladium, Nickel, Kupfer, Mangan und Silber ausgewählt ist, aufweist, wobei die Konzentration an Metall zwischen 5 ppm und 2 Gew.-%, bezogen auf den Katalysator, und das molare Verhältnis $H_2O$/Kohlenwasserstoffe der Charge zwischen 0,1 und 10 liegt.

2. Verfahren nach Anspruch 1, dadurch gekennzeichnet, dass das molare Verhältnis $H_2O$/zu isomerisierende lineare ethylenische Kohlenwasserstoffe zwischen 0,5 und 3 liegt.

3. Verfahren nach Anspruch 2, dadurch gekennzeichnet, dass das molare Verhältnis $H_2O$/zu isomerisierende lineare ethylenische Kohlenwasserstoffe zwischen 0,8 und 2,7 liegt und dass der Katalysator 1 bis 6% Siliciumdioxid aufweist.

4. Verfahren nach einem der Ansprüche 1 bis 3, dadurch gekennzeichnet, dass das Metall aus Palladium besteht und die Konzentration an Palladium zwischen 5 und 100 ppm, bezogen auf den Katalysator, liegt.

5. Verfahren nach Anspruch 4, dadurch gekennzeichnet, dass die Konzentration an Palladium zwischen 10 und 30 ppm liegt.

6. Verfahren nach einem der Ansprüche 1 bis 5, dadurch gekennzeichnet, dass man zwischen 300 und 550° C, unter einem Druck zwischen 0,05 und 1 MPa und mit einer stündlichen Raumgeschwindigkeit zwischen 0,1 und 10, ausgedrückt als Volumen flüssiger Charge pro Volumen Katalysator pro Stunde, arbeitet, und das Verfahren auf die Herstellung von Isobuten durch Behandlung einer Charge, bestehend aus einem Abfluss der katalytischen Krackung, einem Abfluss der Dampfkrakkung, einem Abfluss der Kokung, einem Abfluss der thermischen Krackung, einem Abfluss einer and 3.

8. A process according to Claim 3, wherein the catalyst contains 2 to 4% by weight of silica.

Visbreaking-Operation oder einer Fraktion, die aus einem Abfluss einer Anlage von MTBE (Methyl-tert.-butylether) stammt, anwendet, wobei die Charge mindestens teilweise aus Buten-1 und Buten-2 besteht.

7. Verfahren nach Anspruch 6, dadurch gekennzeichnet, dass man bei einer Temperatur zwischen 400 und 500° C unter einem Druck zwischen 0,08 und 0,4 MPa mit einer stündlichen Raumgeschwindigkeit zwischen 0,5 und 3, ausgedrückt als Volumen der flüssigen Charge pro Volumen Katalysator pro Stunde, arbeitet.

8. Verfahren nach Anspruch 3, dadurch gekennzeichnet, dass der eingesetzte Katalysator 2 bis 4 Gew.-% Siliciumdioxid aufweist.

## Claims

1. A process for isomerizing linear ethylenic hydrocarbons containing 4 to 20 carbon atoms per molecule to branched ethylenic hydrocarbons having the same number of carbon atoms per molecule as the linear hydrocarbons from which they are issued, characterized by the treatment of a hydrocarbons charge consisting of at least a portion of ethylenic linear hydrocarbons, in the presence of steam and of a catalyst containing: (*a*) alumina, (*b*) 0.5 to 10% by weight of silica, and (*c*) at least one metal or metal compound, said metal being selected from the group consisting of chromium, palladium, nickel, copper, manganese and silver, the metal concentration being comprised between 5 ppm and 2% by weight with respect to the catalyst, the molar ratio $H_2O$/hydrocarbons of the charge being from 0.1 to 10.

2. A process according to Claim 1, wherein the molar ratio $H_2O$/linear ethylenic hydrocarbons to isomerize is from 0.5 to 3.

3. A process according to Claim 2, wherein the molar ratio $H_2O$/linear ethylenic hydrocarbons to isomerize is from 0.8 to 2.7 and wherein the catalyst contains 1 to 6% of silica.

4. A process according to one of Claims 1 to 3, wherein the metal is palladium, the palladium concentration being from 5 to 100 ppm, with respect to the catalyst.

5. A process according to Claim 4, wherein said palladium concentration is from 10 to 30 ppm.

6. A process according to one of Claims 1 to 5, conducted at a temperature from 300 to 550°C, under a pressure form 0.05 to 1 MPa and with a space velocity, expressed in volume of liquid charge per volume of catalyst and per hour, comprised between 0.1 and 10, said process being applied to the manufacture of isobutene by treatment of a charge selected from the group consisting of a catalytic cracking effluent, a steam cracking effluent, a coking effluent, a thermal cracking effluent, a visbreaking effluent, and a fraction from an effluent of a MTBE (methyltertiobutylether) producing unit, said charge consisting at least in part of 1-butene and 2-butene.

7. A process according to Claim 6, conducted at a temperature from 400 to 500°C, under a pressure from 0.08 to 0.4 MPa, with a space velocity, expressed in volume of liquid charge per volume of catalyst and per hour, comprised between 0.5